# EUROPÄISCHE PATENTSCHRIFT

(11) **EP 2 743 340 B1**
(45) Veröffentlichungstag und Bekanntmachung des Hinweises auf die Patenterteilung: **29.07.2015**
(21) Anmeldenummer: 13005859.7
(22) Anmeldetag: 17.12.2013
(51) Int. Cl.: C12M 1/107, C12M 1/00, C12M 1/02

(54) **Energieoptimierter Lager- und Fermentationsbehälter für Energieerzeugungs- und Energiespeicheranlagen sowie Verfahren zur Optimierung der Wärmenutzung in einem solchen Behälter**
Energy optimised storage and fermentation vessel for energy generation and energy storage installations and method for optimising the utilisation of heat in such a container
Récipient de stockage et de fermentation optimisé sur le plan énergétique pour système de production d'énergie et d'accumulation d'énergie et procédé d'optimisation de l'utilisation de chaleur dans un tel récipient

(30) Priorität: 17.12.2012 DE 102012024552
(43) Veröffentlichungstag der Anmeldung: 18.06.2014
(73) Patentinhaber: Prüf- und Forschungsinstitut Pirmasens e.V., 66953 Pirmasens (DE)
(72) Erfinder: Pacan, Benjamin, 66957 Trulben (DE); Hell, Stefan, 66271 Kleinblittersdorf (DE)
(74) Vertreter: Patentanwälte Dr. Keller, Schwertfeger

(56) Entgegenhaltungen:
- DD-A3- 256 799
- DE-A1- 10 257 849
- DE-A1-102007 019 159
- DE-A1-102008 048 898
- DE-A1-102009 059 262
- DE-U1-202010 017 546
- FR-A1- 2 481 874
- US-A- 4 350 588
- US-A- 4 780 415

## Beschreibung

Die vorliegende Erfindung betrifft einen energieoptimierten Lager- und Fermentationsbehälter für Energieerzeugungs- und Energiespeicheranlagen gemäß dem Oberbegriff des Anspruchs 1. Die Erfindung betrifft ferner ein Verfahren zur Optimierung der Wärmenutzung in einem Fermentationsbehälter für Energieerzeugungs- und Energiespeicheranlagen.

Biogas wird aus Fermentationsprozessen gewonnen, die in speziell dafür ausgelegten Behältern stattfinden. Bislang wurden für die einzelnen Prozessschritte verschiedene Behälter eingesetzt, in denen die Hydrolyse, die Fermentation, die anschließende Nachgärung sowie die Lagerung der Gärreste sowie der zu fermentierenden Biomasse erfolgten. Solche Behälter sind für die einzelnen Prozessschritte ausgelegt und erfordern in der Ausführung als eigene Lagerbehälter, Fermentationsbehälter sowie Nachgärungsbehälter einen dementsprechend hohen Platzbedarf und aufwändigen Anlagenaufbau. Ein Verfahren und eine Vorrichtung zur Erzeugung von Biogas sind beispielsweise in der WO 2007/068446 beschrieben. Die DE 10 2009 059 262 A1 und die DE 102 57 849 A1 beschreiben Fermentationsbehälter, die beispielsweise für Biogasanlagen geeignet sind.

Das bei der Fermentation entstehende Biogas wird vor der Nutzung in einer Energieerzeugungs- (z.B. einem Blockheizkraftwerk) oder einer Energiespeicheranlage (z.B. Biomethaneinspeisung ins Erdgasnetz) einem Biogasspeicher zugeführt. Der Innenraum eines Biogasbehälters wird zumeist mit einer sogenannten Gasspeicherfolie abgedeckt, unterhalb deren sich der eigentliche Biogasspeicher befindet. Häufig ist die Gasspeicherfolie mittels einer Traglufthaube, welche aus einer festen, witterungsbeständigen Folie besteht, als Schutz gegen Witterungseinflüsse und äußere mechanische Belastungen abgedeckt. Ein solcher Behälter ist beispielsweise in der WO 2005/097629 A1 und der EP 1 882 734 B1 beschrieben. Daneben gibt es Verfahren zur Veredelung von Biogas. Die DE 10 2007 019 159A1 und die DE 198 10 993 A1 beschreiben Verfahren zur Behandlung oder Aufbereitung von Biogas, um beispielsweise Verunreinigungen zu entfernen.

Daneben sind auch Mehrkammersysteme beschrieben, in denen PVC-Wände zur Speicherung von Biogas und eine Gasmembran zur Anwendung kommen. Ein solcher Behälter ist beispielsweise in der EP 0 045 114 A1 beschrieben. In der DE 44 37 717 C1 wird ein Haubenfermenter für ein- und mehrstufige Prozesse vorgeschlagen, der volumenveränderlich ausgebildet und mit einem Ausgleichsbehälter verbunden ist. Dieser Haubenfermenter ist alternativ zur Schaffung einer homogenen Biomasse und zur Trennung von Gasgemischen geeignet. Solche Anlagen werden zur Vergärung von Biomasse, insbesondere von Bioabfällen eingesetzt, um Gärschlamm als Nutzprodukt und Biogas zur energetischen Verwertung zu gewinnen.

Das bei der Fermentation entstehende Biogas besitzt im Wesentlichen dieselbe Temperatur wie der eigentliche Gärprozess. Das Biogas wird in einem Biogasspeicher zwischengelagert und schließlich zur Verbrennung der Energieerzeugungsanlage zugeführt. Die in Form von Wärmeenergie in dem Biogas enthaltene Energie wird bislang energetisch nicht ausgenutzt. Zwar wird in der DE 20 2007 016 024 U1 ein Wärmespeicher beschrieben, dem Wärme aus dem Verbrennungsprozess des Biogases über einen Wärmetauscher zuführbar ist, jedoch wird mit diesem nicht das aus dem Gärprozess gewonnene Biogas behandelt.

Das US 4,780,415 beschreibt ein Verfahren und eine Vorrichtung zum Abbau von organischen Produkten, beispielsweise mittels einer Methanogenese. Die Vorrichtung umfasst mehrere Kammern, ein Gasometer und eine Wärmetauschereinrichtung für das erzeugte Gas.

Vor diesem Hintergrund ist es Aufgabe der vorliegenden Erfindung, einen verbesserten Behälter für Energieerzeugungs- und Energiespeicheranlagen bereit zu stellen, bei dem die einzelnen Prozessschritte der Fermentation, Nachgärung und Lagerung in einem einzelnen Behälter vereint sind und der hinsichtlich der Wärmenutzung optimiert ist.

Diese Aufgabe wird gelöst durch einen energieoptimierten Lager- und Fermentationsbehälter für Energieerzeugungs- und Energiespeicheranlagen mit den Merkmalen des Anspruchs 1.

Bevorzugte Ausführungsformen der vorliegenden Erfindung sind in den Unteransprüchen beansprucht.

Der erfindungsgemäße Lager- und Fermentationsbehälter für Energieerzeugungs- und Energiespeicheranlagen ist bezüglich der Wärmenutzung energieoptimiert und umfasst in seinem Grundaufbau eine Außenwand und einen Boden sowie wenigstens eine Zwischenwand im Inneren des Behälters, die den Behälter in wenigstens zwei Kammern unterteilt. Die einzelnen Kammern können wahlweise zur Hydrolyse, Fermentation, Nachgärung oder Lagerung genutzt werden. Beispielsweise kann in einer Kammer anfänglich Rübenmussilage gelagert werden, welche anschließend in einer benachbarten Kammer hydrolysiert oder fermentiert wird. Dieselbe Kammer kann nach entsprechendem Umpumpen der Biomasse in eine andere Kammer am Ende des Prozesses zur Lagerung von Gärresten genutzt werden. Entsprechend den einzelnen Prozessschritten können nacheinander oder gleichzeitig die Substratlagerung, Hydrolyse, Fermentation, Nachgärung oder Gärrestlagerung erfolgen. Dadurch ist die Kapazität des Behälters mit den einzelnen Kammern durch die vorgesehene Mehrfachnutzung optimal ausgenutzt. Bislang waren für die einzelnen Prozessschritte mehrere separate Behälter notwendig was platzaufwändig und kostenintensiv war. Die einzelnen Prozessschritte können nun mit dem erfindungsgemäßen System in einem einzigen Behälter durchgeführt werden.

Zwischen den einzelnen Kammern des Behälters befindet sich wenigstens eine Überführvorrichtung für die zu verlagernde Biomasse, um den Kammerinhalt von einer Kammer des Behälters in eine benachbarte Kammer zu verlagern. Bei dieser Überführvorrichtung handelt es sich vorzugsweise um eine Pumpe, einen Durchlass oder einen Überlauf. Jedoch sind auch andere Systeme oder Einrichtungen denkbar. Da es sich vorzugsweise um flüssige Biomasse handelt, ist ein Überlauf zwischen den einzelnen Kammern besonders einfach am Kammerboden oder der Kammerwand anzubringen. Zusätzlich kann eine Pumpe oder eine Pumpeinrichtung zum Umpumpen des Kammerinhalts in eine benachbarte Kammer genutzt werden. Somit können mit vergleichsweise geringem Aufwand Umpumpvorgänge innerhalb eines Behälters durchgeführt werden. Ein zusätzlicher freier Durchlass verringert darüber hinaus das notwendige Pumpvolumen. Die Pumpe ist vorzugsweise zwischen den Kammern angeordnet. In einer Ausführungsform ist ein Behälterunterbau vorgesehen, bei dem sich die Pumpstation unterhalb der Kammern befindet.

Der in einer bevorzugten Ausführungsform vorgesehene Behälterunterbau dient zur bedarfsweisen Unterbringung von Pumpen, der Flüssigkeits- und Gaswärmetauscher und/oder der Mess- und Regeltechnik. Mit dem Behälterunterbau können die Leitungslängen für die Flüssigkeits- und Gasleitungen zwischen den einzelnen Kammern reduziert werden, wodurch der erforderliche Flächenbedarf bei Energieerzeugungs- oder Energiespeicheranlagen deutlich gesenkt werden kann.

Je nach Ausführungsform kann der Flächenbedarf für Energieerzeugungs- oder Energiespeicheranlagen bei der erfindungsgemäßen Behältergeometrie gegenüber einer konventionellen Bauweise auf unter 50 % und der Wärmeenergieverlust um mehr als 75 % gesenkt werden. Durch die kompakte, energetisch optimierte Bauweise und die Flüssiglagerung von Substraten können beispielsweise Biogaserzeugungsanlagen optimierter in Industrie- und Gewerbegebiete implementiert und in Verbindung mit Wärmerückgewinnungsanlagen (z.B. Wärmetauscher und Wärmepumpen) auch ohne externer Wärmeenergiezufuhr betrieben werden. Dies stellt einen entscheidenden Vorteil dar.

Zur Speicherung des bei der Fermentation bzw. der Nachgärung entstehenden Biogases ist ein oberhalb der wenigstens zwei Kammern angeordneter und von diesen getrennter Gasspeicherraum zur Zwischenspeicherung des bei der Fermentation und Nachgärung entstehenden Biogases vorgesehen. Der Gasspeicherraum besteht aus einer Decke und einem Boden, wobei im Behälteraufbau der Boden des Gasspeicherraums baubedingt vorzugsweise zugleich auch die Decke der Kammern bildet. Zum Überleiten des bei der Fermentation oder der Nachgärung in einer Kammer entstehenden Biogases in den Gasspeicherraum sind ein in wenigstens einer Kammer angeordneter Gasauslass und entsprechende Leitungen vorgesehen. Vorzugsweise ist der Gasspeicherraum direkt oberhalb der zuvor genannten Kammern angeordnet, so dass ein kompakter Behälter entsteht.

Bisherige Behälter haben zur Speicherung des Biogases die in dem Biogas enthaltene Wärmeenergie entweder nicht oder nur unzureichend genutzt. Hier setzt die vorliegende Erfindung an und sieht nach dem Gasauslass einer Kammer wenigstens eine Wärmetauschereinrichtung für die Kühlung und Entfeuchtung des aus der Kammer über den Gasauslass ausgeleitete Biogas vor. Anschließend ist das über die Wärmetauschereinrichtung heruntergekühlte Gas zur Zwischenspeicherung dem Gasspeicherraum über Leitungen zuführbar. Erfindungsgemäß ist ferner vorgesehen, dass das Behälterdach und der Boden des Gasspeicherraums sowohl gegenüber der Außenwelt als auch den Kammern gasdicht ausgestaltet sind.

Die gasdichte Ausgestaltung des Behälterdaches und des Bodens des Gasspeicherraums erfolgt vorzugsweise mit einer gasdichten Folie (zum Beispiel PVC-Folie) oder einer Gasmembran, so dass das in dem Gasspeicherraum gelagerte Biogas gegenüber der Außenwelt und den darunterliegenden Kammern abgedichtet ist. Zusätzlich kann eine Wärmedämmung erfolgen.

Da die einzelnen Kammern für unterschiedliche oder für dieselben Prozessschritte ausgelegt sind, besitzen diese daher die dafür erforderlichen Ausrüstungen zur Hydrolyse oder Fermentation von Biomasse, zur Nachgärung oder zur Lagerung von Biomasse oder von Gärresten. Beispielsweise sind in einer oder mehreren Kammern Rührwerke zur Umwälzung oder Durchmischung von Biomasse angeordnet. Dazu zählen ferner Regel- und Steuerungssysteme, beispielsweise zur Temperatur- oder Gärkontrolle.

Das Behälterdach des Gasspeicherraums ist in einer Ausführungsform vorzugsweise als Tragluftdach und der Boden als Holzbalkendecke mit gasdichter PE-Folie ausgestaltet. Die Zwischenwände dienen zugleich zur Stützung der Deckenkonstruktion und verringern dadurch den Materialbedarf bei der Herstellung des Behälters. Das Tragluftdach und die Gasspeicherfolie bestehen vorzugsweise aus einer Folie (z.B. Polyestergewebe mit PVC oder aliphatisches TPU als Matrixmaterial), die weitgehend strahlungs- wärmedurchlässig ist. Dadurch kann das über die Wärmetauschereinrichtung heruntergekühlte Biogas durch Strahlung und Umgebungswärme aufgewärmt werden.

Durch die thermische Isolierung des Behälters wird die bei der Fermentation entstehende Abwärme im Behälterinneren gespeichert und kann zusätzlich für den Wärmetausch genutzt werden. Beispielsweise kann die Fermentationswärme das im Gasspeicherraum heruntergekühlte Biogas erneut erwärmen, bevor es über eine Gasreinigung (z. B. Aktivkohlefilter) zur Nutzung einer Energieerzeugungs- oder Energiespeicheranlage zugeführt wird.

Vorzugsweise ist bei der gasdichten Folie oder der Gasmembran in dem Gasspeicherraum ein Kondensatablauf zur Entfeuchtung vorgesehen. Dadurch wird bei schwankenden Tagestemperaturen ohne zusätzlichen Energieaufwand infolge der Kondensatbildung an der Gasmembran eine weitere Entfeuchtung erzielt. Das entstehende Kondensat kann dem Prozess rückgeführt werden. Vorzugsweise fällt der Boden des Gasspeicherraums zur Kondensatsammlung zur Mitte mit einem Winkel von etwa 1 bis 5 %, vorzugsweise um 2,5 % ab.

In dem Gasspeicherraum befindet sich ein Gasauslass zur Überführung des zwischengespeicherten Biogases zu einer energetischen Verwertung (zum Beispiel in einem Blockheizkraftwerk) oder zur Gaskonditionierung und Speicherung in einem Erdgasnetz.

Vorzugsweise ist das Innere des Behälters in drei oder mehr Kammern (zum Beispiel vier oder sechs Kammern) über Zwischenwände aufgeteilt und vorzugsweise sind die Zwischenwände im Inneren des Behälters so angeordnet, dass sie im Zentrum des Behälters aufeinander stoßen. Vorzugsweise werden dabei rechte oder spitze Winkel der Zwischenwände in der Behältermitte angestrebt. Durch diese Geometrie wird die Durchmischbarkeit der Einzelkammern verbessert und damit die Anzahl der erforderlichen Rührwerke reduziert. Vortexerscheinungen, wie sie bei runden Behälterräumen auftreten, werden dadurch vermindert.

Nach einer bevorzugten Ausführungsform sind an der Außenwand des Behälters eine zusätzliche wärmegedämmte Außenschale und am Boden des Gasspeicherraums eine zusätzliche Wärmedämmung ausgebildet. Eine solche optimierte Dämmung vermindert den Wärmeverlust des Behälters und reduziert die erforderliche Heizenergie. Ferner bleiben die im Inneren des Behälters ablaufenden biologischen Prozesse weitgehend stabil, beispielsweise indem die Temperatur konstant gehalten wird. Größere Temperaturschwankungen werden zudem durch die Dämmung gezielt vermieden.

Konstruktiv ist der erfindungsgemäße Behälter in einer bevorzugten Ausführungsform ferner dadurch gekennzeichnet, dass die Außenwand des Behälters mit einer Wärmedämmung und einem Trapezblech zum mechanischen Schutz ausgestaltet ist. Die Zwischenwände des Behälters sind zur statischen Abstützung vorzugsweise mit Edelstahlseilen verbunden, wodurch die Konstruktion deutlich an Stabilität gewinnt. Ferner besitzt der Boden des Behälters vorzugsweise einen Mehrschichtaufbau, bestehend aus wenigstens einer Bodenplatte, einer Wärmedämmung, einer Baufolie, einem Drainvlies, einer Leckagefolie und Schotter. Die Anordnung dieser Komponenten im Mehrschichtaufbau ist je nach Ausführungsform und Bedarf frei wählbar.

In einer besonders bevorzugten Ausführungsform weist die Außengeometrie des Behälters mit den darin angeordneten Kammern einen runden Querschnitt auf, wodurch eine statisch stabile Ausführung des Behälters möglich ist. Ferner werden durch den Mehrkammeraufbau bei gleichem Volumen des Behälters die Herstellungskosten pro Kubikmeter Inhalt gegenüber mehreren kleineren Behältern deutlich reduziert.

Die einzelnen Kammern können durch zusätzliche Zwischenwände, die parallel zur Außenwand verlaufen in weitere Unterkammern aufgeteilt werden. Ferner können im Behälterunterbau noch weitere Kammern für technische Einrichtungen (z.B. Zentralpumpe, Wärmetauscher, Biogasentfeuchter und Biogaskühler etc.) vorgesehen sein. In einer bevorzugten Ausführungsform ist ein Kondenswasserbehälter im Behälterunterbau vorgesehen, der neben dem Kondenswasser des Gasspeicherraums aus der Biogasentfeuchtung auch Regenwasser sammelt. Die Lage der Pumpstation wird vorzugsweise so gewählt, dass die Pumpleitungen für die Biomasse möglichst kurz ausfallen.

Die einzelnen Kammern ermöglichen die Einlagerung unterschiedlicher Medien, die im zeitlichen Verlauf der einzelnen Prozessschritte gewechselt werden. Lagerung, Hydrolyse, Fermentation und Nachgärprozesse können somit in unterschiedlichen Kammern hintereinander oder gleichzeitig erfolgen, so dass der vorhandene Platz im Inneren des Behälters unter Anpassung an die Situation optimal ausgenutzt werden kann. Ferner ermöglicht der erfindungsgemäße Aufbau des Behälters durch Trennung der Kammern von dem Gasspeicherraum eine Abtrennung des warmen Bereiches in den Kammern von dem kühleren Bereich des zwischengespeicherten Biogases in dem Gasspeicherraum. Durch den Aufbau wird die wärmeübertragende Oberfläche deutlich reduziert, damit verringern sich auch Wärmeverluste deutlich. Die optimierte Dämmung des Behälters vermindert den Wärmeverlust und reduziert dadurch auch die zur Aufrechterhaltung des Fermentationsprozesses erforderliche Heizenergie.

In einer besonders bevorzugten Ausführungsform umfasst der erfindungsgemäße Behälter mehr als eine Wärmetauschereinrichtung. Die Anlage kann beispielsweise eine oder mehrere Wärmepumpen, eine oder mehrere Kälteadsorptionsmaschinen und/oder eine oder mehrere Wärmetauscher umfassen. Bevorzugt ist wenigstens eine Wärmepumpe für den Gasauslass nach der Kammer. Mit solchen Einrichtungen wird eine optimale Wärmenutzung erreicht. In einem solchen Behälter wird das im Fermenter gebildete Biogas über eine erste Wärmetauschereinrichtung (z.B. Kälteadsorptionsmaschine) auf eine Temperatur heruntergekühlt, bei der eine Entfeuchtung des Biogases erfolgt (z.B. bei einer Temperatur von ca. 5°C). Das so entfeuchte Biogas wird in den Gasspeicherraum geleitet. Hier kann in einer Warmperiode (z.B. im Sommer) eine Erwärmung über das strahlungs- und wärmedurchlässige Tragluftdach des Behälters erfolgen. Ansonsten wird das Biogas über eine weitere zweite Wärmetauschereinrichtung (Wärmetauscher) auf die gewünschte Temperatur erwärmt und der weiteren Biogasverwertung zugeführt.

In einer Kaltperiode (z.B. im Winter), bei der die Außentemperaturen geringer als 10°C sind und genügend Heizenergie zur Verfügung steht, kann die Abkühlung und Entfeuchtung des Biogases auch direkt über den Gasspeicherraum erfolgen. Das Kondensat wird anschließend in einen Kältespeicher geleitet. Vorzugsweise umfasst der Behälter je nach Ausbaustufe zusätzlich einen oder mehrere Wärmespeicher, einen oder mehrere Heizwasserspeicher und/oder einen oder mehrere Kältespeicher.

Die Abwärme der Adsorptionskältemaschine (z.B. mit einer Temperatur von ca. 45°C) kann über eine dritte Wärmetauschereinrichtung (Wärmetauscher) in einem Wärmespeicher gespeichert werden. Ein ebenfalls vorgesehener Heizwasserspeicher wird vorzugsweise von der Hydrolysestufe und/oder über eine Wärmepumpe aus dem Wärmespeicher geheizt. Vorzugsweise wird der Fermenter über eine große ungedämmte Wandfläche direkt beheizt. Über eine weitere Wärmepumpe kann Heißwasser für den Betrieb der Kälteadsorptionsmaschine und zur Einleitung von Überschüssen ins Nahwärmenetz erzeugt werden.

Die Erfindung umfasst auch ein Verfahren, bei dem die oben beschriebenen Prozessschritte und Maßnahmen zur Energieoptimierung in einem Lager- und Fermentationsbehälter umgesetzt werden. Die Verfahrensabläufe werden anhand der oben beschriebenen Erläuterungen und den Figurenbeschreibungen ersichtlich.

Das erfindungsgemäße Verfahren zur Optimierung der Wärmenutzung in einem Fermentationsbehälter für Energieerzeugungs- und Energiespeicheranlagen ist dadurch gekennzeichnet, dass das bei der Fermentation entstehende Biogas zunächst über wenigstens eine Wärmetauschereinrichtung abgekühlt und entfeuchtet und anschließend einem gasdichten Gasspeicherraum zur Zwischenspeicherung zugeführt wird. Vorzugsweise erfolgt über zusätzliche Wärmetauschereinrichtungen bedarfsweise eine Entfeuchtung und Abkühlung oder Erwärmung des Biogases für die entsprechenden Nutzungszwecke, wobei eine Energiespeicherung und Energienutzung über einen oder mehrere Wärmespeicher, einen oder mehrere Heizwasserspeicher und/oder einen oder mehrere Kältespeicher erfolgt. Die in der Fermentationskammer gebildete Fermentationswärme zur Erwärmung des im Gasspeicherraums heruntergekühlten Biogases genutzt wird.

Vorzugsweise ist vorgesehen, dass das Biogas über einen zweiten Wärmetauscher auf eine gewünschte Temperatur erwärmt und der Biogasverwertung zugeführt wird.

Vorzugsweise ist vorgesehen, dass die Abkühlung und Entfeuchtung des Biogases direkt über den Gasspeicherraum erfolgt und das Kondensat anschließend in einen Kältespeicher geleitet wird.

Die Erfindung wird in den nachfolgenden Zeichnungen näher erläutert.

In Fig. 1 ist ein erfindungsgemäßer energieoptimierter Lager- und Fermentationsbehälter für Energieerzeugungs- und Energiespeicheranlagen in einem Vier-Kammer-Aufbau gezeigt.

In Fig. 2 ist ein erfindungsgemäßer energieoptimierter Lager- und Fermentationsbehälter für Energieerzeugungs- und Energiespeicheranlagen in einem Sechs-Kammer-Aufbau gezeigt.

In Fig. 3 ist ein erfindungsgemäßer energieoptimierter Lager- und Fermentationsbehälter mit Behälterunterbau gezeigt.

In Fig. 4 ein erfindungsgemäßer energieoptimierter Lager- und Fermentationsbehälter mit zusätzlichen Einrichtungen zum Betrieb gezeigt.

In Fig. 5 ist ein erfindungsgemäßer energieoptimierter Lager- und Fermentationsbehälter mit mehreren Wärmetauschereinrichtungen zur Wärmenutzung gezeigt.

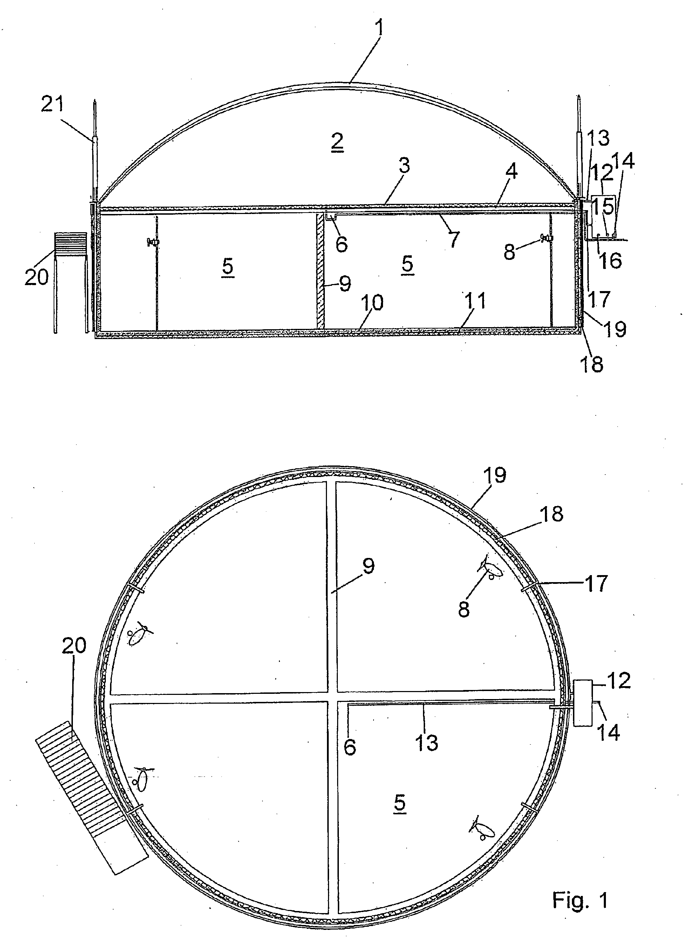

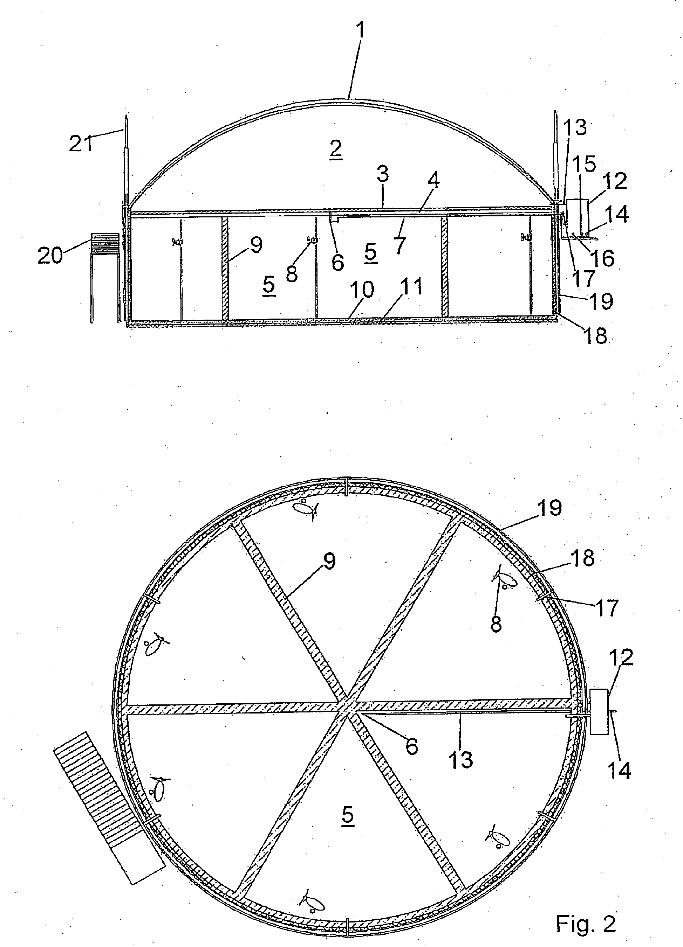

In Fig. 1 ist ein erfindungsgemäßer energieoptimierter Lager- und Fermentationsbehälter gezeigt. Der Behälter besteht aus einer Außenwand 19 sowie einem Boden 10. Das Innere des Behälters wird durch mehrere Zwischenwände 9 in vier Kammern 5 eingeteilt. Als Boden 10 dient eine STB-Bodenplatte. Zusätzlich ist ein Mehrschichtaufbau 11 vorgesehen, bestehend aus Wärmedämmung, Baufolie, Drainvlies, Leckagefolie und Schotter. In den Kammern 5 sind Rührwerke 8 zur Umwälzung der Biomasse zu erkennen. Je nach Nutzung können die Kammern 5 entweder zur Lagerung von frischer Biomasse, zur Hydrolyse, zur Fermentation, zur Nachgärung oder zur Lagerung von Gärresten verwendet werden. Zwischen den Kammern 5 befinden sich Überführvorrichtungen 23, um den Kammerinhalt von einer Kammer 5 des Behälters in eine benachbarte Kammer 5 zu verlagern (siehe Figuren 2 ff.). Bei der Überführeinrichtung 23 handelt es sich vorzugsweise um eine Pumpe, einen Durchlass oder einen Überlauf (nicht gezeigt).

Oberhalb der Kammern 5 ist ein Gasspeicherraum 2 angeordnet, der aus einem Behälterdach 1 und einem Boden 4 besteht. Der Boden 4 besteht aus Holzbalken, PE-Folie sowie einer zusätzlichen Wärmedämmung. Diese Komponenten bilden die Isolierschicht 3. Vorzugsweise fällt der Boden 4 mit der gasdichten Folie zur Mitte mit einem Winkel von etwa 2,5 % ab, damit sich Kondensat in einem Siphon 6 sammeln kann, welches über Leitungen 7 einem Kondensatablauf zugeführt wird.

Durch die Fermentation und bei sehr niedrigen Außentemperaturen durch externe Wärmezufuhr über einen Wärmetauscher wird die Biomasse erwärmt, wobei das während der Fermentation oder Nachgärung entstehende Biogas in etwa dieselbe Temperatur aufweist. Um diesen Temperaturunterschied zu nutzen, ist eine Wärmepumpe 12 vorgesehen, mit der das aus den Kammern 5 über einen Gasauslass 17 entweichende Gas durch das Wärmepumpenprinzip abzukühlen (z. B. Adsorptionskälte). Die Wärmepumpe als Wärmetauschereinrichtung 12 besitzt zusätzlich einen Abwärmevorlauf 15 sowie einen Abwärmerücklauf 16. Anschließend wird das heruntergekühlte Gas dem Gasspeicherraum 2 zugeführt. Über einen Gasausgang 13 kann das auf diese Weise zwischengespeicherte Gas der Energieerzeugungs- oder Energiespeicheranlage zugeführt werden. Im Vergleich zu herkömmlichen Anlagen wird das Biogas im Gasspeicherraum 2 deshalb mit weit weniger Temperatur gespeichert, als dies bislang der Fall war. Zusätzliche Maßnahmen wie Gasabdichtung und Wärmedämmung sorgen ebenfalls für eine höhere Energieeffizienz.

Die Außenwand 19 besitzt eine wärmegedämmte Außenschale 18, bestehend aus Wandelementen sowie Polystyrolhartschaumplatten. Die Außenwand 19 ist in der gezeigten Ausführungsform aus Trapezblech hergestellt. Daneben kann der Behälter noch mit den üblichen Blitzschutzeinrichtungen 21 sowie einer Arbeitsbühne 20 ausgestattet werden.

Der Grundriss des erfindungsgemäßen Behälters ist in der gezeigten Ausführungsform rundförmig, wobei zu erkennen ist, dass die Zwischenwände 9 in der Mitte mit einem rechten Winkel aufeinander stoßen. Dies hat den Vorteil, dass die Durchmischbarkeit der einzelnen Kammern 5 durch die eckige Geometrie verbessert wird und damit die Anzahl der Rührwerke reduziert. Die Rührwerke in einem runden Behälter führen regelmäßig zu vortexartigen Verwirbelungen. Bei einem Behälter mit mehr als vier Kammern treffen die Zwischenwände 9 in einem spitzen Winkel aufeinander (vgl. Fig. 2).

In Fig. 2 ist eine weitere Ausführungsform des erfindungsgemäßen Behälters gezeigt. Dieser besteht aus sechs Kammern, die zur Hydrolyse oder Fermentation, zur Nachgärung oder zur Lagerung verwendet werden können. Die Zwischenwände 9 laufen zur Mitte in einem spitzen Winkel zueinander. Die sonstigen Einrichtungen entsprechen denen wie bereits in der Fig. 1 beschrieben.

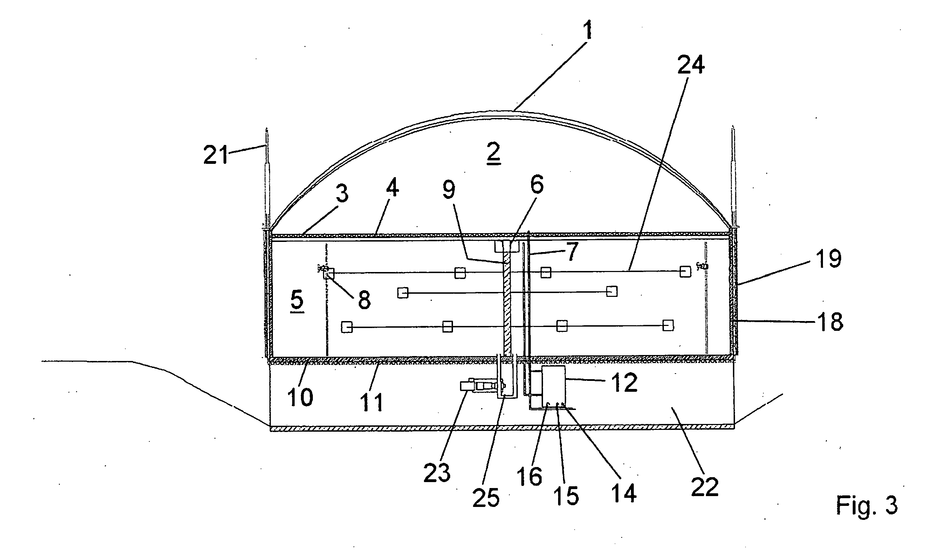

In Fig. 3 ist eine weitere Ausführungsform des erfindungsgemäßen Behälters mit einem Behälterunterbau 22 gezeigt. Im Behälterunterbau 22 befinden sich in der gezeigten Ausführungsform wenigstens eine Pumpe als Überführeinrichtung 23 und die Wärmetauschereinrichtung 12 mit dem Abwärmerücklauf 16, Abwärmevorlauf 15 und Kondensatablauf 14. Das Kondensat des Gasspeicherraums 2 wird im Siphon 6 gesammelt und über eine Leitung 7 der Wärmetauschereinrichtung 12 zugeführt. Die flüssige Biomasse wird über die Pumpe 23 über entsprechend ausgelegte Leitungen 25 zwischen den Kammern 5 hin und her gepumpt. Durch die flächensparende Installation der Pumptechnik, der Flüssigkeits- und Gaswärmetauscher sowie der Mess- und Regeltechnik können Umpumpvorgänge flexibler mit einer einzigen Pumpe 23 realisiert werden und die Leitungslängen für Flüssigkeits- und Gasrohre stark reduziert werden. Die Innenwände des Behälters werden mit Halteseilen 24 zusätzlich gesichert.

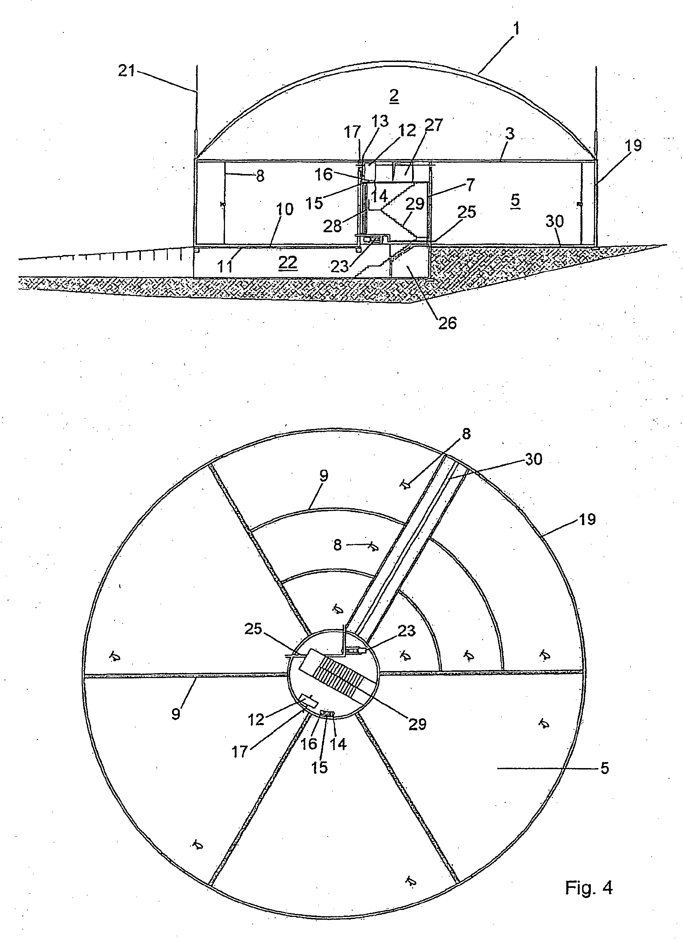

In Fig. 4 ist eine weitere Ausführungsform des erfindungsgemäßen Behälters gezeigt. Hierbei befindet sich im Behälterunterbau 22 ein Kondenswasserbehälter 26, in dem sich Kondenswasser aus der Entfeuchtung des Biogases sammelt. Das Kondenswasser wird über eine Leitung 7 dem Kondenswasserbehälter 26 zugeführt. Gleichzeit führt auch eine Regenwasserleitung 30 zum Kondenswasserbehälter 26, so dass das Wasser gesammelt werden kann. Oberhalb befindet sich eine Pumpstation mit einer Pumpe 23, um den Kammerinhalt einer Kammer 5 in eine andere benachbarte Kammer 5 über Leitungen 25 zu pumpen. Unterhalb der Wärmetauschereinrichtung 12 befindet sich ein Wärmetauscher 28 für das Substrat. Über Treppen 29 sind innerhalb des Behälters die einzelnen Einrichtungen erreichbar. Über einen Wartungstunnel 27 können Instandhaltungsnahmen vorgenommen werden. Über Bullaugen in den Zwischenwänden 9 können zudem Anzeigen und Geräte überwacht werden.

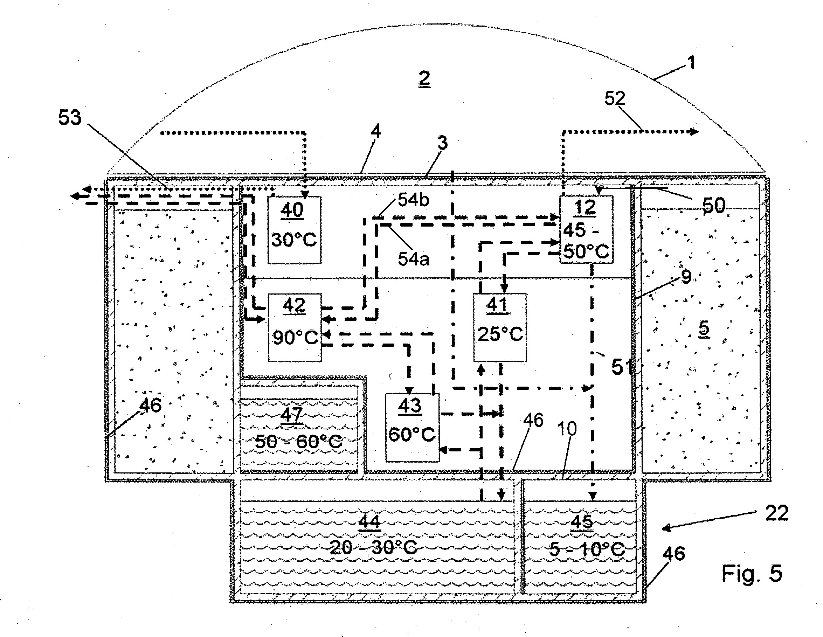

In Fig. 5 ist eine Ausführungsform des Behälters mit mehreren Wärmetauschereinrichtungen gezeigt. Bei dem gezeigten Behälter sind mehrere Wärmetauschereinrichtungen 12, 40, 41, 42 und ein Heizwasserspeicher 43 vorgesehen. Unterhalb des Behälterbodens 10 befindet sich ein Behälterunterbau 22. In diesem ist ein Warmwasserspeicher als Wärmespeicher 44 sowie ein über eine Zwischenwand von diesem getrennter Kaltwasserspeicher 45 angeordnet. Die Temperatur des Wärmespeichers 44 beträgt in der gezeigten Ausführungsform etwa 20 bis 30°C. Der Kaltwasserspeicher 45 besitzt eine Temperatur von etwa 5 bis 10°C. Daneben ist noch ein Heißwasserspeicher 47 mit einer Temperatur von etwa 50 bis 60°C vorgesehen. Das in der Fermentationskammer 5 gebildete Biogas (mit einer Temperatur zwischen 37 und 45°C) wird als feuchtes, warmes Biogas 50 einer Kälteadsorptionsmaschine als Wärmetauschereinrichtung 12 zugeführt und auf etwa 5°C abgekühlt. Das bei Entfeuchtung entstehende Kondensat 51 wird dem Kaltwasserspeicher 45 zugeführt. Das entfeuchtete und heruntergekühlte Biogas 52 wird anschließend dem Gasspeicherraum 2 zugeführt und in diesem zwischengespeichert.

In der gezeigten Ausführungsform befindet sich am Boden 3 des Gasspeicherraums 2 sowie am Behälterdach 1 eine Folie, die weitgehend strahlungs- und wärmedurchlässig ist, wodurch sich das auf etwa 5°C abgekühlte Biogas 52 durch Strahlungs- sowie Umgebungswärme (aus der Fermentation) aufgewärmt wird. Das Biogas 52 kann in dem Gasspeicherraum 2 zirkulieren und beispielsweise über das andere Ende des Gasspeicherraums 2 über einen ersten Wärmetauscher 40 auf die gewünschte Temperatur (beispielsweise 30°C) erwärmt werden. Anschließend kann das so erwärmte Biogas 53 einer Biogasverwertungsanlage zugeführt werden.

In einer Kaltperiode (beispielsweise bei Temperaturen unterhalb von 10°C), in der genügend Heizenergie zur Verfügung steht, kann die Abkühlung und Entfeuchtung des Biogases 50 auch direkt über den Gasspeicherraum 2 erfolgen (nicht gezeigt). Anschließend wird das Kondensat 51, wie oben beschrieben, in den Kältespeicher 45 geleitet.

Ferner ist vorgesehen, dass die Abwärme der Adsorptionskältemaschine (beispielsweise 45°C) über eine zweite Wärmetauschereinrichtung 41 erwärmt und in einem Wärmespeicher 44 mit bei Temperatur von 20 bis 30°C gespeichert wird. Ein zusätzlicher Heißwasserspeicher 47 mit einer Temperatur von etwa 50 bis 60°C wird von der Hydrolysestufe sowie über eine erste Wärmepumpe 43 (ca. 60°C) geheizt. Über eine zweite Wärmepumpe 42 kann Heißwasser (ca. 80 bis 90°C) für den Betrieb der Kälteadsorptionsmaschine 12 erzeugt werden. Das Heißwasser kann ferner zur Einleitung von Überschüssen in das nahe Wärmenetz eingesetzt werden. Die einzelnen Wärmetauschereinrichtungen 12, 40, 41, 42 besitzen jeweils wenigstens einen Wasservorlauf und/oder Wasserrücklauf 54a, 54b.

Die einzelnen Kammern 5 und die Speicherräume 44, 45, 47 sind in der gezeigten Ausführungsform mit einer Wärmedämmung 46 versehen. Auch der Boden des Gasspeicherraums 2 besitzt eine Wärmedämmung 3.

Der erfindungsgemäße Behälter zeichnet sich somit dadurch aus, dass zum einen die einzelnen Prozessschritte in mehrfach genutzten Behälterkammern ausgeführt werden und dass zudem eine Wärmeenergieoptimierung bei dem entstehenden Biogas über die Wärmetauschermaßnahmen erfolgt.

## Patentansprüche

1. Energieoptimierter Lager- und Fermentationsbehälter für Energieerzeugungs- und Energiespeicheranlagen, umfassend
- eine Außenwand (19),
- einen Boden (10),
- wenigstens eine Zwischenwand (9) im Inneren des Behälters, die den Behälter in wenigstens zwei Kammern (5) unterteilt, die wahlweise zur Hydrolyse oder Fermentation von Biomasse, zur Nachgärung oder zur Lagerung von Biomasse und von Gärresten ausgerüstet sind, wobei zwischen den einzelnen Kammern (5) eine Überführeinrichtung (23) für die Biomasse vorgesehen ist, um den Kammerinhalt von einer Kammer (5) des Behälters in eine benachbarte Kammer (5) zu verlagern,
- einen oberhalb der wenigstens zwei Kammern (5) angeordneten und von diesen getrennten Gasspeicherraum (2) zur Zwischenspeicherung des bei der Fermentation und Nachgärung entstehenden Biogases, wobei der Gasspeicherraum (2) aus einem Behälterdach (1) und einem Boden (4) besteht,
und das Behälterdach (1) und der Boden (4) des Gasspeicherraums (2) gasdicht ausgestaltet sind,
- einen in wenigstens einer Kammer (5) angeordneten Gasauslass (17) zum Überleiten des bei der Fermentation oder der Nachgärung in einer Kammer (5) entstehenden Biogases in den Gasspeicherraum (2),
**dadurch gekennzeichnet, dass**
- nach dem Gasauslass (17) einer Kammer (5) wenigstens eine Wärmetauschereinrichtung (12) zur Entfeuchtung und Abkühlung für das aus der Kammer (5) über den Gasauslass (17) ausgeleitete Biogas angeordnet ist,
- Leitungen zum Zuführen des über die wenigstens eine Wärmetauschereinrichtung (12) behandelten Gases in den Gasspeicherraum (2) vorgesehen sind.

2. Behälter nach Anspruch 1, **dadurch gekennzeichnet, dass** das Behälterdach (1) und/oder der Boden (4) des Gasspeicherraums (2) mit einer gasdichten Folie oder einer Gasmembran abgedichtet ist/sind und dass die Folie des Behälterdaches (1) des Gasspeicherraums (2) ferner strahlungs- und wärmedurchlässig ist.

3. Behälter nach einem der vorhergehenden Ansprüche, **dadurch gekennzeichnet, dass** der Boden (4) des Gasspeicherraums (2) zugleich die Decke der wenigstens zwei Kammern (5) bildet.

4. Behälter nach einem der vorhergehenden Ansprüche, **dadurch gekennzeichnet, dass** der Boden (4) des Gasspeicherraums (2) zur Mitte mit einem Winkel von etwa 1 bis 5 % abfällt und am Boden (4) ein Kondensatablauf angeordnet ist.

5. Behälter nach einem der vorhergehenden Ansprüche, **dadurch gekennzeichnet, dass** unterhalb des Behälterbodens (10) ein zusätzlicher Behälterunterbau (22) angeordnet ist.

6. Behälter nach einem der vorhergehenden Ansprüche, **dadurch gekennzeichnet, dass** an der Außenwand (19) des Behälters eine zusätzliche wärmegedämmte Außenschale (18) und am Boden (4) des Gasspeicherraums (2) eine zusätzliche Wärmedämmung (3) ausgebildet sind.

7. Behälter nach einem der vorhergehenden Ansprüche, **dadurch gekennzeichnet, dass** die wenigstens eine Wärmetauschereinrichtung (12) mit einem Abwärmerücklauf (16), einem Abwärmevorlauf (15) und einem Kondensatablauf (14) ausgerüstet ist.

8. Behälter nach einem der vorhergehenden Ansprüche, **dadurch gekennzeichnet, dass** der Behälter mehrere Wärmetauschereinrichtungen (12, 40, 41, 42) umfasst, ausgewählt aus der Gruppe bestehend aus Kälteadsorptionsmaschine (12), Wärmetauscher (40, 41) und Wärmepumpe (42), die derart angeordnet und verbunden sind, dass eine Abkühlung und Entfeuchtung und bedarfsweise eine Erwärmung des Biogases für die entsprechenden Nutzzwecke erfolgen kann.

9. Behälter nach einem der vorhergehenden Ansprüche, **dadurch gekennzeichnet, dass** der Behälter einen oder mehrere Wärmespeicher (44), einen oder mehrere Heizwasserspeicher (43) und/oder einen oder mehrere Kältespeicher (45) umfasst, wobei in dem Kältespeicher (45) das bei der Entfeuchtung bei der Wärmetauschereinrichtung (12) entstehende Kondensatz (51) zuführbar ist.

10. Behälter nach einem der vorhergehenden Ansprüche, **dadurch gekennzeichnet, dass** es sich bei der Überführeinrichtung (23) der Kammern (5) um eine Pumpe, einen Durchlass oder einen Überlauf handelt.

11. Verfahren zur Optimierung der Wärmenutzung in einem Fermentationsbehälter für Energieerzeugungs- und Energiespeicheranlagen, wobei der Fermentationsbehälter folgende Merkmale umfasst:
- eine Außenwand (19), einen Boden (10) und wenigstens eine Zwischenwand (9) im Inneren des Behälters, die den Behälter in wenigstens zwei Kammern (5) unterteilt, die wahlweise zur Hydrolyse oder Fermentation von Biomasse, zur Nachgärung oder zur Lagerung von Biomasse und von Gärresten genutzt werden, wobei der Kammerinhalt von einer Kammer (5) des Behälters in eine benachbarte Kammer (5) über eine zwischen den Kammern (5) angeordnete Überführeinrichtung (23) verlagert wird,
- einen oberhalb der wenigstens zwei Kammern (5) angeordneten und von diesen getrennten Gasspeicherraüm (2) zur Zwischenspeicherung des bei der Fermentation und Nachgärung entstehenden Biogases, wobei der Gasspeicherraum (2) aus einem Behälterdach (1) und einem Boden (4) besteht, und das Behälterdach (1) und der Boden (4) des Gasspeicherraums (2) gasdicht ausgestaltet sind,
- einen in wenigstens einer Kammer (5) angeordneten Gasauslass (17) zum Überleiten des bei der Fermentation oder der Nachgärung in einer Kammer (5) entstehenden Biogases in den Gasspeicherraum (2),
**dadurch gekennzeichnet, dass**
- das aus der Kammer (5) über den Gasauslass (17) ausgeleitete Biogas über wenigstens eine nach dem Gasauslass (17) einer Kammer (5) angeordneten Wärmetauschereinrichtung (12) entfeuchtet und abgekühlt wird,
- das über die wenigstens eine Wärmetauschereinrichtung (12) behandelte Gas in den gasdichten Gasspeicherraum (2) geleitet wird.

12. Verfahren nach Anspruch 11, **dadurch gekennzeichnet, dass** über zusätzliche Wärmetauschereinrichtungen (40, 41, 42) bedarfsweise eine Entfeuchtung und Abkühlung oder Erwärmung des Biogases für die entsprechenden Nutzungszwecke erfolgt.

13. Verfahren nach Anspruch 11 oder 12, **dadurch gekennzeichnet, dass** die in der Fermentationskammer gebildete Fermentationswärme zur Erwärmung des im Gasspeicherraum (2) heruntergekühlten Biogases genutzt wird.

14. Verfahren nach einem der Ansprüche 11 bis 13, **dadurch gekennzeichnet, dass** das Biogas über eine- zweite Wärmetauschereinrichtung (41) auf eine gewünschte Temperatur erwärmt und der Biogasverwertung zugeführt wird.

15. Verfahren nach einem der Ansprüche 11 bis 14, **dadurch gekennzeichnet, dass** die Abkühlung und Entfeuchtung des Biogases direkt über den Gasspeicherraum (2) erfolgt und das Kondensat anschließend in einen Kältespeicher (45) geleitet wird.

## Claims

1. Energy-optimised storage and fermentation tank for energy production installations and energy storage installations, comprising
- an outer wall (19),
- a base (10),
- at least one intermediate wall (9) which is located inside the tank and divides the tank into at least two chambers (5), which are optionally equipped for hydrolysis or fermentation of biomass, for secondary fermentation, or for storage of biomass and digestate, a transfer device (23) for the biomass being provided between the individual chambers (5) for moving the contents of one chamber (5) of the tank into an adjacent chamber (5),
- a gas storage volume (2) which is arranged above the at least two chambers (5) and separated therefrom and which is intended for temporarily storing the biogas produced during fermentation and secondary fermentation, the gas storage volume (2) consisting of a tank roof (1) and a base (4), and the tank roof (1) and the base (4) of the gas storage volume (2) being gas-tight,
- a gas outlet (17) arranged in at least one chamber (5) for conducting the biogas, produced in one chamber (5) during fermentation or secondary fermentation, into the gas storage volume (2),
**characterised in that**
- at least one heat exchanger device (12) for dehumidifying and cooling the biogas conducted out of the chamber (5) via the gas outlet (17) is arranged downstream of the gas outlet (17) of one chamber (5),
- lines are provided for feeding the gas which has been treated by means of the at least one heat exchanger device (12) to the gas storage volume (2).

2. Tank according to claim 1, **characterised in that** the tank roof (1) and/or the base (4) of the gas storage volume (2) is/are sealed by a gas-tight film or a gas membrane, and **in that** the film of the tank roof (1) of the gas storage volume (2) is also permeable to radiation and heat.

3. Tank according to either of the preceding claims, **characterised in that** the base (4) of the gas storage volume (2) simultaneously forms the ceiling of the at least two chambers (5).

4. Tank according to any of the preceding claims, **characterised in that** the base (4) of the gas storage volume (2) tapers towards the centre at an angle of from approximately 1 to 5 % and a condensate drainage line is arranged in the base (4).

5. Tank according to any of the preceding claims, **characterised in that** an additional tank substructure (22) is arranged below the tank base (10).

6. Tank according to any of the preceding claims, **characterised in that** an additional heat-insulating outer shell (18) is arranged on the outer wall (19) of the tank and additional heat insulation (3) is formed on the base (4) of the gas storage volume (2).

7. Tank according to any of the preceding claims, **characterised in that** the at least one heat exchanger device (12) is equipped with a waste heat recirculation line (16), a waste heat supply line (15) and a condensate drainage line (14).

8. Tank according to any of the preceding claims, **characterised in that** the tank comprises a plurality of heat exchanger devices (12, 40, 41, 42), selected from the group consisting of cold adsorption machines (12), heat exchangers (40, 41) and heat pumps (42), which are arranged and connected such that the biogas can be cooled, dehumidified and, if required, heated for the relevant intended use.

9. Tank according to any of the preceding claims, **characterised in that** the tank comprises one or more heat accumulators (44), one or more hot water reservoirs (43) and/or one or more cold accumulators (45), it being possible to feed the condensate (51) which is produced during dehumidification in the heat exchanger device (12) to the cold accumulator (45).

10. Tank according to any of the preceding claims, **characterised in that** the transfer device (23) of the chambers (5) is a pump, a passage or an overflow.

11. Method for optimising the utilisation of heat in a fermentation tank for energy production installations and energy storage installations, the fermentation tank comprising the following features:
- an outer wall (19), a base (10) and at least one intermediate wall (9) which is located inside the tank and divides the tank into at least two chambers (5), which are optionally used for hydrolysis or fermentation of biomass, for secondary fermentation, or for storage of biomass and digestate, the contents of one chamber (5) of the tank being moved into an adjacent chamber (5) by means of a transfer device (23) arranged between the chambers (5),
- a gas storage volume (2) which is arranged above the at least two chambers (5) and separated therefrom and which is intended for temporarily storing the biogas produced during fermentation and secondary fermentation, the gas storage volume (2) consisting of a tank roof (1) and a base (4), and the tank roof (1) and the base (4) of the gas storage volume (2) being gas-tight,
- a gas outlet (17) arranged in at least one chamber (5) for conducting the biogas, produced in one chamber (5) during fermentation or secondary fermentation, into the gas storage volume (2),
**characterised in that**
- the biogas conducted out of the chamber (5) via the gas outlet (17) is dehumidified and cooled by means of at least one heat exchanger device (12) arranged downstream of the gas outlet (17) of one chamber (5),
- the gas treated by means of at least one heat exchanger device (12) is conducted into the gas-tight gas storage volume (2).

12. Method according to claim 11, **characterised in that** the biogas is dehumidified, cooled or heated as required for the relevant intended use by means of additional heat exchanger devices (40, 41, 42).

13. Method according to either claim 11 or claim 12, **characterised in that** the fermentation heat created in the fermentation chamber is used to heat the biogas that has been cooled down in the gas storage volume (2).

14. Method according to any of claims 11 to 13, **characterised in that** the biogas is heated to a desired temperature by means of a second heat exchanger device (41) and fed to the biogas utilisation system.

15. Method according to any of claims 11 to 14, **characterised in that** the biogas is cooled and dehumidified directly by means of the gas storage volume (2) and the condensate is then conducted into a cold accumulator (45).

## Revendications

1. Récipient de stockage et de fermentation optimisé sur le plan énergétique pour systèmes de production d'énergie et d'accumulation d'énergie, comprenant
- une paroi extérieure (19),
- un fond (10),
- au moins une paroi intermédiaire (9) à l'intérieur du récipient, qui divise le récipient en au moins deux chambres (5) qui sont équipées au choix pour l'hydrolyse ou la fermentation de biomasse, pour la post-fermentation ou pour le stockage de biomasse et de digestats, un dispositif de transfert (23) pour la biomasse étant prévu entre les différentes chambres (5) pour déplacer le contenu d'une chambre (5) du récipient dans une chambre (5) voisine,
- un espace de stockage de gaz (2) disposé au-dessus desdites au moins deux chambres (5) et séparé de celles-ci pour le stockage intermédiaire du biogaz produit lors de la fermentation et de la post-fermentation, l'espace de stockage de gaz (2) étant composé d'un toit de récipient (1) et d'un fond (4), le toit de récipient (1) et le fond (4) de l'espace de stockage de gaz (2) étant conçus étanches au gaz,
- une sortie de gaz (17) disposée dans au moins une chambre (5) pour faire passer le biogaz produit dans une chambre (5) lors de la fermentation ou de la post-fermentation dans l'espace de stockage de gaz (2),
**caractérisé en ce que**
- au moins un dispositif d'échange de chaleur (12) pour la déshumidification et le refroidissement du biogaz évacué de la chambre (5) par la sortie de gaz (17) est disposé après la sortie de gaz (17) d'une chambre (5),
- des conduites sont prévues pour amener le gaz traité par ledit au moins un dispositif d'échange de chaleur (12) dans l'espace de stockage de gaz (2).

2. Récipient selon la revendication 1, **caractérisé en ce que** le toit de récipient (1) et/ou le fond (4) de l'espace de stockage de gaz (2) est/sont rendu(s) étanche(s) par une feuille étanche au gaz ou une membrane à gaz et que la feuille du toit de récipient (1) de l'espace de stockage de gaz (2) est en outre perméable au rayonnement et à la chaleur.

3. Récipient selon l'une des revendications précédentes, **caractérisé en ce que** le fond (4) de l'espace de stockage de gaz (2) forme en même temps le plafond desdites au moins deux chambres (5).

4. Récipient selon l'une des revendications précédentes, **caractérisé en ce que** le fond (4) de l'espace de stockage de gaz (2) est en pente vers le centre d'un angle d'environ 1 à 5 % et une évacuation du condensat est disposée sur le fond (4).

5. Récipient selon l'une des revendications précédentes, **caractérisé en ce qu'**une infrastructure de récipient (22) supplémentaire est disposée sous le fond de récipient (10).

6. Récipient selon l'une des revendications précédentes, **caractérisé en ce qu'**une enveloppe extérieure supplémentaire isolée thermiquement (18) est formée sur la paroi extérieure (19) du récipient et une isolation thermique supplémentaire (3) sur le fond (4) de l'espace de stockage de gaz (2).

7. Récipient selon l'une des revendications précédentes, **caractérisé en ce que** ledit au moins un dispositif d'échange de chaleur (12) est équipé d'un retour de chaleur perdue (16), d'un départ de chaleur perdue (15) et d'une évacuation de condensat (14).

8. Récipient selon l'une des revendications précédentes, **caractérisé en ce que** le récipient comprend plusieurs dispositifs d'échange de chaleur (12, 40, 41, 42), sélectionnés dans le groupe constitué d'une machine frigorifique à adsorption (12), d'un échangeur de chaleur (40, 41) et d'une pompe à chaleur (42) qui sont disposés et reliés de façon qu'un refroidissement et une déshumidification et, si nécessaire, un réchauffement du biogaz puissent avoir lieu aux fins d'usage correspondantes.

9. Récipient selon l'une des revendications précédentes, **caractérisé en ce que** le récipient comprend un ou plusieurs accumulateurs de chaleur (44), un ou plusieurs réservoirs d'eau chaude (43) et/ou un ou plusieurs accumulateurs de froid (45), le condensat (51) produit lors de la déshumidification dans le dispositif d'échange de chaleur (12) pouvant être amené dans l'accumulateur de froid (45).

10. Récipient selon l'une des revendications précédentes, **caractérisé en ce que** le dispositif de transfert (23) des chambres (5) est une pompe, un passage ou un déversoir.

11. Procédé pour optimiser l'utilisation de la chaleur dans un récipient de fermentation pour systèmes de production d'énergie et d'accumulation d'énergie, le récipient de fermentation présentant les caractéristiques suivantes :
- une paroi extérieure (19), un fond (10) et au moins une paroi intermédiaire (9) à l'intérieur du récipient, qui divise le récipient en au moins deux chambres (5) qui sont utilisées au choix pour l'hydrolyse ou la fermentation de biomasse, pour la post-fermentation ou pour le stockage de biomasse et de digestats, le contenu d'une chambre (5) du récipient étant déplacé dans une chambre (5) voisine par un dispositif de transfert (23) disposé entre les chambres (5),
- un espace de stockage de gaz (2) disposé au-dessus desdites au moins deux chambres (5) et séparé de celles-ci pour le stockage intermédiaire du biogaz produit lors de la fermentation et de la post-fermentation, l'espace de stockage de gaz (2) étant composé d'un toit de récipient (1) et d'un fond (4), le toit de récipient (1) et le fond (4) de l'espace de stockage de gaz (2) étant conçus étanches au gaz,
- une sortie de gaz (17) disposée dans au moins une chambre (5) pour faire passer le biogaz produit dans une chambre (5) lors de la fermentation ou de la post-fermentation dans l'espace de stockage de gaz (2),
**caractérisé en ce que**
- le biogaz évacué de la chambre (5) par la sortie de gaz (17) est déshumidifié et refroidi par au moins un dispositif d'échange de chaleur (12) disposé après la sortie de gaz (17) d'une chambre (5),
- le gaz traité par ledit au moins un dispositif d'échange de chaleur (12) est conduit dans l'espace de stockage de gaz (2) étanche au gaz.

12. Procédé selon la revendication 11, **caractérisé en ce qu'**une déshumidification et un refroidissement ou un réchauffement du biogaz aux fins d'usage correspondantes sont effectués si nécessaire au moyen de dispositifs d'échange de chaleur supplémentaires (40, 41, 42).

13. Procédé selon la revendication 11 ou 12, **caractérisé en ce que** la chaleur de fermentation formée dans la chambre de fermentation est utilisée pour réchauffer le biogaz refroidi dans l'espace de stockage de gaz (2).

14. Procédé selon l'une des revendications 11 à 13, **caractérisé en ce que** le biogaz est réchauffé à une température souhaitée par un deuxième dispositif d'échange de chaleur (41) et amené à la valorisation du biogaz.

15. Procédé selon l'une des revendications 11 à 14, **caractérisé en ce que** le refroidissement et la déshumidification du biogaz sont réalisés directement par l'espace de stockage de gaz (2) et le condensat est ensuite conduit dans un accumulateur de froid (45).
